# EUROPEAN PATENT APPLICATION

(11) **EP 4 790 461 A1**
(43) Date of publication of application: **12.08.2026**
(21) Application number: 26154047.0
(22) Date of filing: 26.01.2026
(51) Int. Cl.: G01T 1/20

(54) **IMAGING PLATE AND IMAGE SCANNER**

(30) Priority: 06.02.2025 JP 2025018077
(71) Applicant: J. MORITA MANUFACTURING CORPORATION, Fushimi-ku, Kyoto-shi, Kyoto 6128533 (JP)
(72) Inventor: FUJII, Yusuke, Kyoto, 612-8533 (JP); SUGIHARA, Yoshito, Kyoto, 612-8533 (JP); YOSHIOKA, Tadashi, Kyoto, 612-8533 (JP); YASUI, Kazuhiro, Kyoto, 612-8533 (JP)
(74) Representative: Müller Hoffmann & Partner

(57) **Abstract**

An object is to provide: an imaging plate on which information can be recorded without reducing a region in which a radiographic image is recorded; and an image scanner used for the imaging plate. An imaging plate according to the present disclosure is an imaging plate on which a radiographic image is recorded, and includes: a substrate; a radiographic image forming layer formed on the substrate, the radiographic image forming layer accumulating energy of a radiation irradiated, the radiographic image forming layer being configured to emit emission light according to the energy accumulated; and a protective layer formed on the radiographic image forming layer to protect the radiographic image forming layer. The protective layer has: a first reflective area having a surface on which light is received and reflected in a first direction; and a second reflective area having a surface on which the light is received and reflected in a second direction different from the first direction, and prescribed information is recorded in the protective layer by a pattern formed in the second reflective area.

## Description

### REFERENCE TO RELATED APPLICATIONS

This application is based on Japanese Patent Application No. 2025-018077 filed on February 6, 2025 with the Japan Patent Office.

### BACKGROUND

### Field

The present disclosure relates to an imaging plate and an image scanner.

### Description of the Background Art

In dentistry, intraoral imaging has been known as one of methods of radiographing the inside of an oral cavity. In intraoral imaging, in the state in which an imaging plate on which an X-ray image (a radiographic image) can be recorded is held inside a mouth, X-rays are applied from outside toward teeth behind of which the imaging plate is located. In this way, radiography is performed (Japanese Patent Laying-Open No. 2023-76397).

### SUMMARY OF THE INVENTION

After an X-ray image recorded on the imaging plate is read by an image scanner, the recorded X-ray image is erased from the imaging plate by an erasing light source, so that the imaging plate is reused. However, since the imaging plate deteriorates every time it is used, the number of uses of the imaging plate needs to be managed. In dental clinics, a plurality of imaging plates are often equipped, and thus, individual imaging plates need to be identified and managed.

However, if identification information for identifying each imaging plate is recorded on a back surface of the imaging plate, a dedicated image scanner is required that is provided with a sensor or the like for reading the information on the back surface of the imaging plate. Further, if identification information for identifying the imaging plate is recorded on a front surface of the imaging plate, the region in which an X-ray image is recorded is reduced by the region in which the identification information is recorded.

The present disclosure has been made in view of the above-described problem, and an object thereof is to provide: an imaging plate on which information can be recorded without reducing a region in which a radiographic image is recorded; and an image scanner used for the imaging plate.

An imaging plate according to the present disclosure is an imaging plate on which a radiographic image is recorded, and includes: a substrate; a radiographic image forming layer formed on the substrate, the radiographic image forming layer accumulating energy of a radiation irradiated, the radiographic image forming layer being configured to emit emission light according to the energy accumulated; and a protective layer formed on the radiographic image forming layer to protect the radiographic image forming layer. The protective layer has: a first reflective area having a surface on which light is received and reflected in a first direction; and a second reflective area having a surface on which the light is received and reflected in a second direction different from the first direction. Prescribed information is recorded in the protective layer by a pattern formed in the second reflective area.

An image scanner according to the present disclosure is an image scanner that reads a radiographic image from the imaging plate, and includes: a light source configured to irradiate the imaging plate with excitation light; a first detector configured to receive photostimulable light generated from the imaging plate upon receipt of the excitation light emitted from the light source; a second detector configured to receive the excitation light reflected in the second reflective area; and a circuit configured to read, from the photostimulable light received by the first detector, a radiographic image recorded on the imaging plate. The circuit is configured to read, from the excitation light reflected in the second reflective area and received by the second detector, the prescribed information recorded in the protective layer.

The foregoing and other objects, features, aspects, and advantages of the present disclosure will become apparent from the following detailed description of the present disclosure, which is understood in conjunction with the accompanying drawings.

### BRIEF DESCRIPTION OF THE DRAWINGS

Fig. 1A is a plan view showing a configuration of an imaging plate according to an embodiment.
Fig. 1B is a cross-sectional view showing the configuration of the imaging plate according to the embodiment.
Fig. 2 is a diagram for illustrating intraoral imaging using the imaging plate.
Fig. 3 is a schematic diagram of an image scanner for reading the imaging plate.
Fig. 4 is a diagram showing an example of a radiographic image recorded on the imaging plate.
Fig. 5A is a diagram for illustrating reflection of light received on a surface in a first reflective area of the imaging plate.
Fig. 5B is a diagram for illustrating reflection of the light received on the surface in the first reflective area of the imaging plate.
Fig. 6 is a diagram for illustrating reflection of light received on a surface in a second reflective area of the imaging plate.
Fig. 7A is a diagram for illustrating a process of reading the recorded radiographic image and prescribed information from the imaging plate by the image scanner according to the embodiment.
Fig. 7B is a diagram for illustrating the process of reading the recorded radiographic image and the prescribed information from the imaging plate by the image scanner according to the embodiment.
Fig. 7C is a diagram for illustrating the process of reading the recorded radiographic image and the prescribed information from the imaging plate by the image scanner according to the embodiment.
Fig. 8 is a diagram for illustrating image processing on an image obtained by reading the recorded radiographic image from the imaging plate.
Fig. 9A is a schematic diagram showing a modification of the second reflective area of the imaging plate.
Fig. 9B is a schematic diagram showing a modification of the second reflective area of the imaging plate.
Fig. 9C is a schematic diagram showing a modification of the second reflective area of the imaging plate.
Fig. 10 is a schematic diagram showing a modification of the image scanner for reading the imaging plate.
Fig. 11 shows the laser marking device 300.

### DESCRIPTION OF THE PREFERRED EMBODIMENTS

Hereinafter, an embodiment of the present disclosure will be described with reference to the drawings.

### <Embodiment>

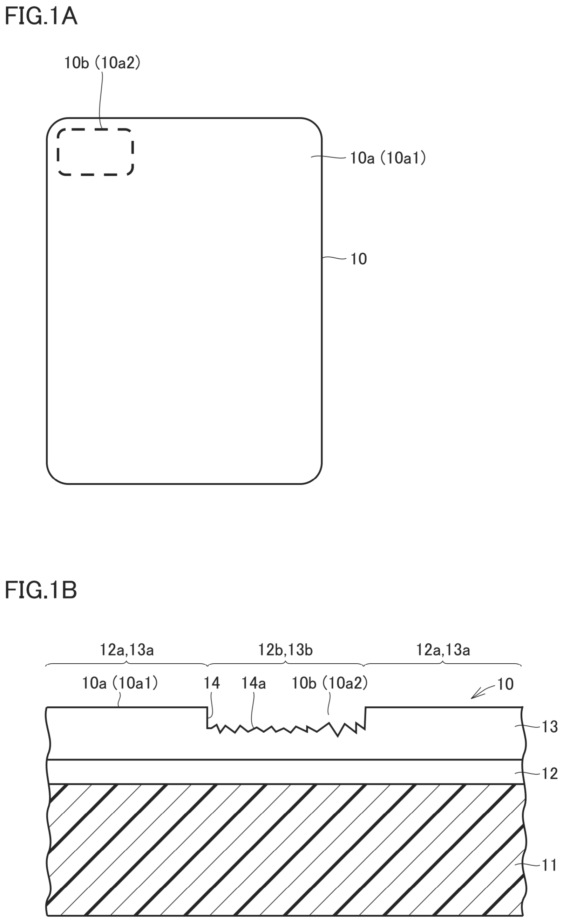

First, a configuration of an imaging plate according to an embodiment of the present disclosure will be described. Fig. 1A is a plan view showing a configuration of an imaging plate 10 according to the embodiment. Fig. 1B is a cross-sectional view showing the configuration of imaging plate 10 according to the embodiment. Imaging plate 10 has a flat shape having a radiographic image forming layer 12, and serves as a recording medium on which a radiographic image is recorded. Imaging plate 10 has, for example, a substantially rectangular flat shape.

Imaging plate 10 has a substrate 11 (a film) formed of a resin and having one main surface coated with a photostimulable phosphor serving as radiographic image forming layer 12. Further, imaging plate 10 has a protective layer 13 formed on radiographic image forming layer 12 for protecting radiographic image forming layer 12. Protective layer 13 is a resin (for example, polyethylene terephthalate (PET)) transparent to visible light or X-rays. Radiographic image forming layer 12 is a layer in which energy of an applied radiation is accumulated and which serves to emit emission light according to the accumulated energy, i.e., photostimulable light in this case. As a radiation applied to radiographic image forming layer 12, for example, X-rays are employed. Regarding the arrangement of radiographic image forming layer 12 and substrate 11, it is assumed that radiographic image forming layer 12 is defined as a front layer and substrate 11 is defined as a back layer. In this case, regarding the front and back surfaces of imaging plate 10, the surface on the front layer side may be considered as a "front surface", and the surface on the back layer side may be considered as a "back surface". In the present embodiment, the surface of protective layer 13 is a "front surface".

In imaging plate 10, when X-rays from an X-ray generator are applied and pass through an object to be photographed, energy corresponding to the intensity of the X-rays is accumulated in radiographic image forming layer 12. Since the intensity of the X-rays is based on the distribution of the X-ray absorption region in the object to be photographed, the distribution of the energy accumulated in radiographic image forming layer 12 shows a radiographic image of the object to be photographed, which is formed by X-rays. In this way, for example, a radiographic image formed by X-rays can be recorded as a latent image on imaging plate 10.

Further, as shown in Fig. 1A, imaging plate 10 has, on its front surface side, a recording region 10a in which a radiographic image is recorded and an information region 10b in which identification information of imaging plate 10 is recorded. As shown in Fig. 1B, in information region 10b, at least one recess 14 is provided in the surface of protective layer 13, and the identification information of imaging plate 10 is recorded by the pattern formed by recess 14. Herein, a portion of protective layer 13 that forms recording region 10a is referred to as a recording region protective layer 13a, and a portion of protective layer 13 that forms information region 10b is referred to as an information region protective layer 13b. Further, a portion of radiographic image forming layer 12 that forms recording region 10a is referred to as a radiographic image forming layer recording region 12a, and a portion of radiographic image forming layer 12 that forms information region 10b is referred to as a radiographic image forming layer information region 12b. Merely by having recess 14 in which identification information of imaging plate 10 is recorded, information region protective layer 13b has properties of transparency that allow excitation light to reach radiographic image forming layer 12 (radiographic image forming layer information region 12b) located under information region protective layer 13b. Thus, as in recording region 10a, excitation light is applied to radiographic image forming layer 12 (radiographic image forming layer information region 12b) located under information region protective layer 13b, and then, a radiographic image can be read out based on the photostimulable light emitted from the recorded radiographic image. In this case, since information region 10b has a function of recording a radiographic image, information region 10b can also be considered as a recording region in that sense. Accordingly, recording region 10a may be considered as a "first recording region 10al", and information region 10b may be considered as a "second recording region 10a2". Recording region protective layer 13a may be considered as "first recording region protective layer 13a", and information region protective layer 13b may be considered as "second recording region protective layer 13b". Further, radiographic image forming layer recording region 12a may be considered as a "first radiographic image forming layer", and radiographic image forming layer information region 12b may be considered as a "second radiographic image forming layer". When a certain substance allows excitation light L1 to pass therethrough, this substance is expressed as being transparent to excitation light, and when a certain substance allows photostimulable light to pass therethrough, this substance is expressed as being transparent to photostimulable light. In such a case, protective layer 13 is transparent both to excitation light and photostimulable light in each of recording region protective layer 13a and information region protective layer 13b.

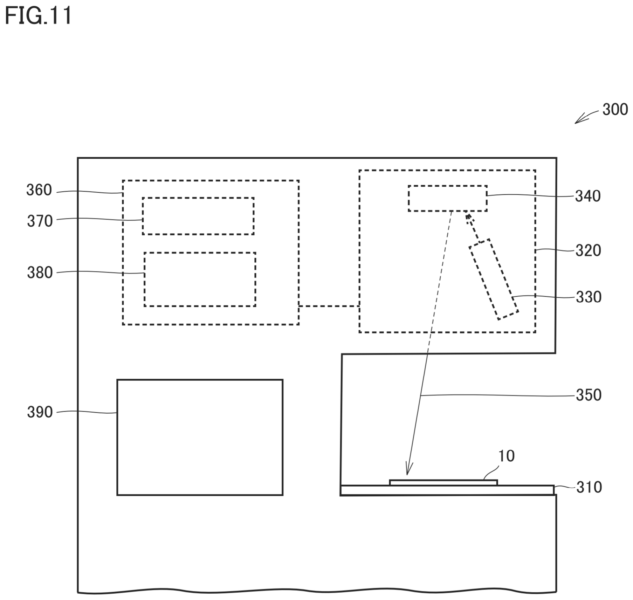

Recess 14 is formed in the surface of protective layer 13, for example, by a laser marking device. Fig. 11 shows the laser marking device 300. The laser marking device comprises a supporter 310 which supports imaging plate 10, a laser irradiator 320, a control unit which is circuitry implemented by a circuit unit 360, an operation interface 390. The laser irradiator 320 comprises a laser source 330, an irradiation direction changing element 340. The circuit unit 360 is configured, for example, by a computer circuit including at least one processor 370 and a storage unit 380. The processor is a central processing unit (CPU) or the like, and is configured by an electric circuit, the circuit unit 360 can be regarded as a laser irradiation circuitry. The circuit unit 360 controls the laser irradiator 320. By executing a irradiation program by the processor 370, various functions for irradiating are implemented. The circuit unit 360 can be called as a irradiation control circuitry. A laser light as an action laser beam emitted from laser source 330 is incident on irradiation direction changing element 340, and then reflected by irradiation direction changing element 340 to reach protective layer 13 of the imaging plate 10. According to the irradiation program the irradiating direction of the action laser beam 350 is changed.

The laser marking device 300 shaves the surface of protective layer 13 with laser light (action laser light 350) to form recess 14, and then, forms a pattern such as a barcode or a two-dimensional code indicating identification information of imaging plate 10 on the surface of protective layer 13. Thus, as shown in Fig. 1B, a bottom surface 14a of recess 14 is rougher than the surface of protective layer 13 (the surface of recording region 10a), and reflects the received light in a direction different from the direction in which the light received on the surface of protective layer 13 is reflected. Or the laser marking device 300 shaves the surface of protective layer 13 so that the bottom surface 14a of recess 14 is rougher than the surface of protective layer 13. In other words, imaging plate 10 has recording region 10a (a first reflective area) and information region 10b (a second reflective area) as regions in which rays of light received on their surfaces are reflected in different directions. Specifically, protective layer 13 has: the first reflective area in which the light received on its surface is reflected in a first direction; and the second reflective area in which the light received on its surface is reflected in a second direction different from the first direction. Recording region protective layer 13a forms the first reflective area, and information region protective layer 13b forms the second reflective area. In imaging plate 10 according to the present embodiment, by using the regions in which rays of light are reflected in different directions, the identification information of imaging plate 10 can be recorded without reducing the region in which a radiographic image is recorded, as described later.

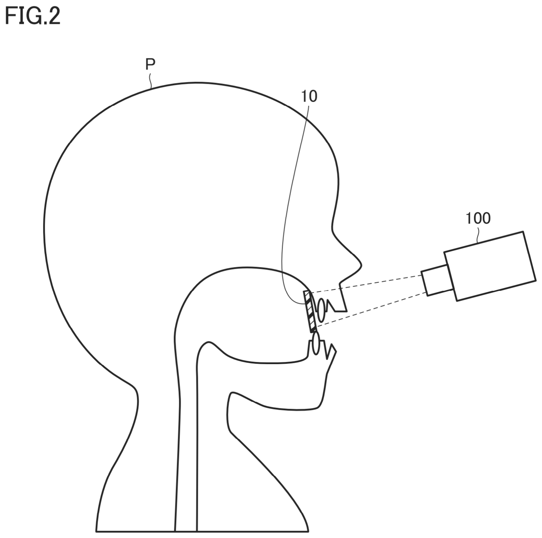

In the present embodiment, imaging plate 10 is irradiated with a radiation, for example, in the state in which imaging plate 10 is placed inside a patient's mouth. Fig. 2 is a diagram for illustrating intraoral imaging using imaging plate 10. In intraoral imaging, in the state in which imaging plate 10 capable of recording a radiographic image (an X-ray image) is held inside a mouth such that the front surface of imaging plate 10 faces toward teeth and the back surface thereof faces toward a throat, an X-ray generator 100 applies X-rays from outside toward the teeth behind of which imaging plate 10 is located, so that radiography is performed. Thus, imaging plate 10 is sized such that it can be placed inside a mouth of a patient P. Then, a radiographic image of the teeth is recorded in radiographic image forming layer 12 of imaging plate 10. Note that the use of imaging plate 10 is not limited thereto.

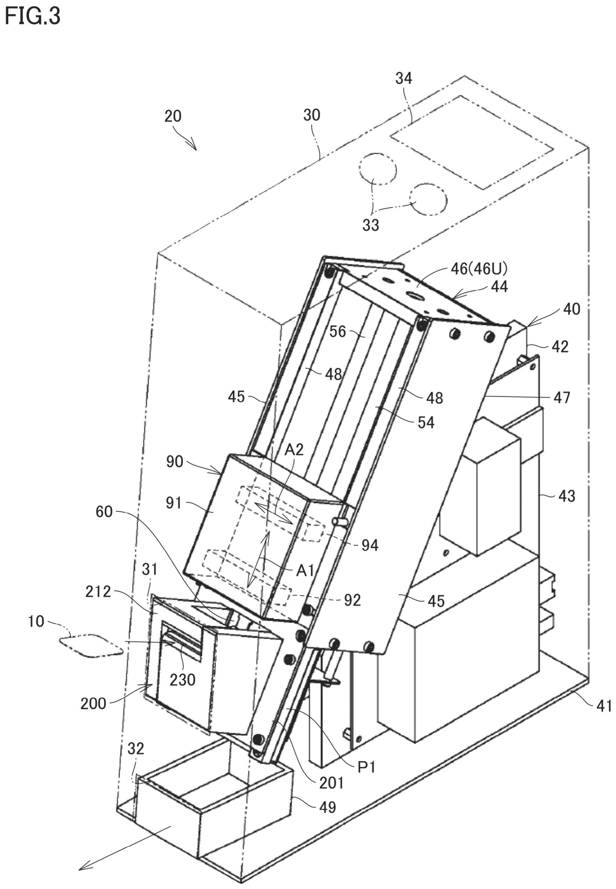

The radiographic image recorded in radiographic image forming layer 12 of imaging plate 10 is read by an image scanner. Fig. 3 is a schematic diagram of the image scanner for reading the imaging plate. An image scanner 20 reads a radiographic image from radiographic image forming layer 12 and generates an image signal (also referred to as image data) representing the read radiographic image. Image scanner 20 includes a housing 30. Inside housing 30, a stage 60, an excitation light source 92, a light detector 94, and a setting guide 200 are provided. Stage 60 supports imaging plate 10 on the side of substrate 11 (on the back surface side). Imaging plate 10 supported by stage 60 is irradiated with excitation light from excitation light source 92. When imaging plate 10 is irradiated with excitation light, radiographic image forming layer 12 of imaging plate 10 emits light. This emission light (photostimulable light) is detected by light detector 94. Based on the signal detected by light detector 94, image data of a radiographic image is generated.

Housing 30 has an opening 31, setting guide 200 is fixed to housing 30 so as to be accommodated in opening 31, and a slot 230 of setting guide 200 is exposed to the outside through opening 31. Setting guide 200 serves to guide, toward stage 60, imaging plate 10 supplied from the outside of image scanner 20. Unlike setting guide 200 located at a fixed position with respect to housing 30, stage 60 is movable in a main scanning direction A1 inside housing 30. This configuration will be described later. Setting guide 200 includes a guide base plate 201 and a guide body 212. When stage 60 is located at a set position P1, a user of image scanner 20 inserts imaging plate 10 through slot 230 into setting guide 200, so that imaging plate 10 can be set on stage 60 in a posture suitable to be read.

An outlet port 32 is provided in a lower portion of housing 30, and a collection tray 49 is disposed in outlet port 32. Imaging plate 10 discharged from stage 60 is collected in collection tray 49. The user of image scanner 20 can pull out collection tray 49 in outlet port 32 to the outside, and collect imaging plate 10 that has been read.

Housing 30 is provided with a switch 33 for receiving various instructions. Switch 33 is, for example, a power switch, a start switch for instructing start of reading, or the like. Further, housing 30 is provided with a display device 34. Display device 34 is, for example, a liquid crystal display panel or an organic electro-luminescence (EL) display panel. Display device 34 can display, for example, a radiographic image that has been read. Display device 34 may be a touch panel having a function of detecting a touch operation.

Stage 60, excitation light source 92, light detector 94, and setting guide 200 are supported by a support member 40 provided inside housing 30. Support member 40 includes a base plate 41, an intermediate support plate 42, and a box-shaped portion 44. Box-shaped portion 44 includes a pair of long-side side plates 45, a pair of short-side side plates 46, and a back plate 47. A pair of support rods 48 are supported at edges of the pair of long-side side plates 45 on the side opposite to back plate 47 such that the pair of support rods 48 are in postures inclined with respect to the direction of gravity.

Excitation light source 92 is a laser light source that emits laser light as excitation light and emits, for example, visible laser light, which may be red laser light or may be laser light of a different color. Light detector 94 serves as a sensor that detects light emitted by excitation light from imaging plate 10 and outputs a signal corresponding to the intensity of the detected light. Light detector 94 may have a configuration in which elements for detecting light are arranged in a line. For example, light detector 94 may be disposed in a posture in which the elements are arranged in a direction parallel to a sub-scanning direction A2. Each of the elements for detecting light may be a silicon photomultiplier tube, a photomultiplier tube, a photodiode, and the like.

Excitation light source 92 and light detector 94 are integrated with each other as a reading unit 90. For example, excitation light source 92 and light detector 94 are integrated with each other in the state in which they are accommodated in a module case 91. When a motor (not shown) is driven, a screw shaft portion 54 rotates to thereby cause stage 60 to move in main scanning direction A1 along a guide rod 56. During movement of stage 60 in main scanning direction A1, the laser light from excitation light source 92 is incident on a surface of radiographic image forming layer 12 of imaging plate 10 held by stage 60, and the destination to which the laser light is applied moves in sub-scanning direction A2. Thereby, the surface of radiographic image forming layer 12 sequentially emits rays of emission light along a line extending in sub-scanning direction A2.

Light detector 94 is provided at a position where it can detect the emission light generated by the laser light from excitation light source 92 and emitted from radiographic image forming layer 12. For example, excitation light source 92 is disposed so as to irradiate imaging plate 10 with laser light from an oblique direction, and light detector 94 is disposed in front of a position at which imaging plate 10 is irradiated with laser light. Then, when the surface of radiographic image forming layer 12 sequentially emits rays of emission light along a line extending in sub-scanning direction A2, the emission light is detected by light detector 94. Light detector 94 detects, for example, the intensity of the emission light at a certain point (an irradiation point) and immediately thereafter detects the intensity of the emission light at another point (an irradiation point) located next to this certain point in sub-scanning direction A2. When detection in sub-scanning direction A2 completes in a certain row and detection shifts to another row located next to this certain row in main scanning direction A1 (movement in a column direction), then, detection in sub-scanning direction A2 is newly performed. This process is repeatedly performed to obtain intensity information broadening in a two-dimensional matrix in sub-scanning direction A2 and main scanning direction A1.

The operation of stage 60 is controlled by a control unit. The control unit controls the direction and the amount of rotation of the motor to thereby control the movement of stage 60 in main scanning direction A1. The control unit is circuitry implemented by a circuit unit 43. Circuit unit 43 is configured, for example, by a computer including at least one processor and a storage unit. The processor is a central processing unit (CPU) or the like, and is configured by an electric circuit. By executing a reading program by the processor, various functions for reading are implemented. Circuit unit 43 also implements an image processing unit that reads, from the photostimulable light received by light detector 94, a radiographic image recorded on imaging plate 10.

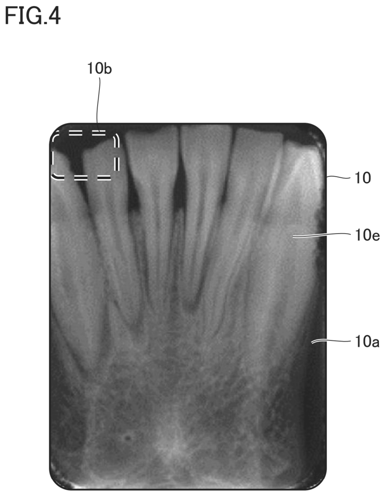

Fig. 4 is a diagram showing an example of a radiographic image recorded on imaging plate 10. Pieces of the intensity information obtained by scanning and broadening in a two-dimensional matrix are distributed into arrangements corresponding to respective detected positions and are reconstructed in the arrangements. In recording region 10a shown in Fig. 4, a radiographic image 10e is shown. Radiographic image 10e is shown also in information region 10b. Fig. 4 shows an area surrounded by a dashed line for indicating information region 10b in order to easily recognize the position of information region 10b, but the area surrounded by the dashed line is not shown enough to be easily visually distinguishable in an actual radiographic image recorded on imaging plate 10, and thus, the user cannot visually recognize information region 10b.

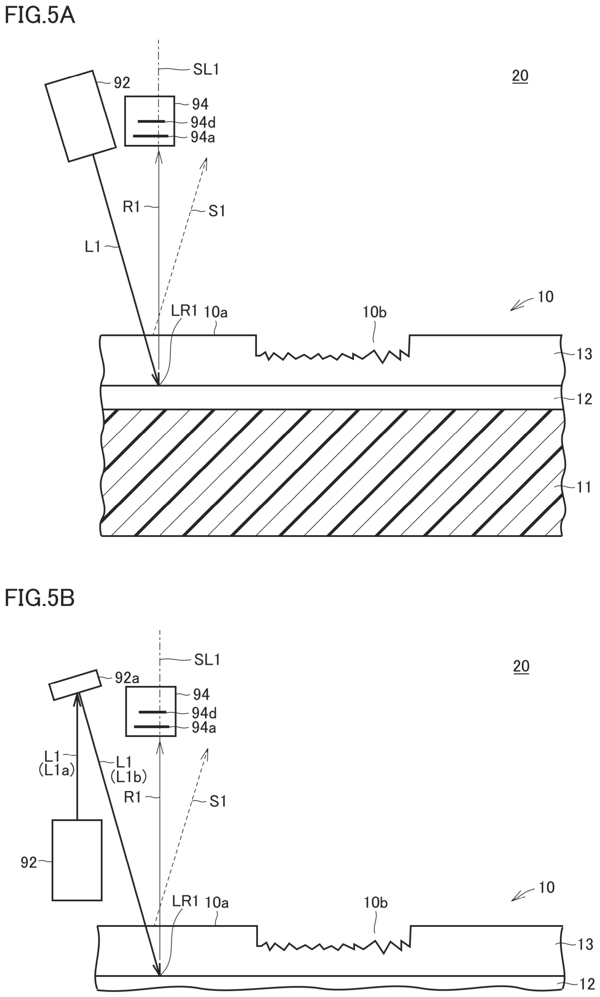

The following specifically describes a principle and a method of reading out the identification information of imaging plate 10 recorded in information region 10b. Figs. 5A and 5B are diagrams each for illustrating reflection of light received on the surface in the first reflective area (recording region 10a) of imaging plate 10. Image scanner 20 shown in Fig. 5A irradiates imaging plate 10 with excitation light L1 from excitation light source 92 in order to read the radiographic image recorded on imaging plate 10 as described above. By excitation light L1 that has passed through protective layer 13 of imaging plate 10 and reached the surface of radiographic image forming layer 12, photostimulable light R1 is emitted based on the radiographic image recorded in radiographic image forming layer 12. By detecting photostimulable light R1, light detector 94 can read a radiographic image.

Excitation light L1 with which imaging plate 10 is irradiated also includes light that is reflected on the surface of protective layer 13 in addition to light that passes through protective layer 13 of imaging plate 10 and reaches the surface of radiographic image forming layer 12. However, reflected light S1 reflected on the surface of protective layer 13 (recording region protective layer 13a) is reflected in a direction in which it cannot be detected by light detector 94. Thus, as shown in Figs. 5A and 5B, in recording region 10a, light detector 94 can detect only photostimulable light R1 but cannot detect reflected light S1. Light detector 94 is provided with an optical filter 94a through which rays of light having wavelengths of photostimulable light R1 and reflected light S1 can pass.

Photostimulable light R1 is detected on a detection surface 94d of light detector 94. It is assumed that an irradiation point LR1 denotes a point on radiographic image forming layer 12 at which excitation light L1 is received. It is assumed that a straight line SL1 denotes a geometrically determined straight line perpendicular to the surface of radiographic image forming layer 12 at irradiation point LR1. Light detector 94 is arranged to be spaced apart from the front surface of imaging plate 10 in a direction in which the front surface of imaging plate 10 faces, and such that detection surface 94d is located on straight line SL1. This arrangement is suitable because the light condensing efficiency for photostimulable light R1 is high in this arrangement. Then, excitation light source 92 is arranged to be spaced apart from the front surface of imaging plate 10 in a direction in which the front surface of imaging plate 10 faces, is located at a position not overlapping with straight line SL1, and is configured such that excitation light L1 is applied in a direction in which irradiation point LR1 is irradiated therewith. Further, excitation light L1 is set to be applied in a direction in which reflected light S1 reflected at irradiation point LR1 in recording region 10a (the first reflective area) of radiographic image forming layer 12 does not hit detection surface 94d. It is assumed that the direction parallel to sub-scanning direction A2 is referred to as a forward sub-scanning direction. In this case, reading unit 90 is configured such that the same arrangement relation among excitation light source 92, irradiation point LR1, and light detector 94 as described above is maintained at any point on radiographic image forming layer 12 in a view along the forward sub-scanning direction.

In other words, excitation light source 92 and light detector 94 are configured in the following manner.
- Light detector 94 is disposed such that detection surface 94d of light detector 94 is located on geometrically determined straight line SL1 perpendicular to the surface of radiographic image forming layer 12 at irradiation point LR1 on the surface of radiographic image forming layer 12 at which excitation light L1 emitted from excitation light source 92 is applied.
- Excitation light source 92 is disposed at a position not overlapping with straight line SL1, and is configured such that excitation light L1 is applied in a direction in which reflected light S1 reflected at irradiation point LR1 in recording region 10a (the first reflective area) does not hit detection surface 94d of light detector 94.

In the present disclosure, as shown in Fig. 5A, excitation light source 92 is mainly arranged directly at a starting point of excitation light L1, but this arrangement is merely for facilitating understanding of principles. Thus, in practice, as shown in Fig. 5B, an irradiation direction changing element 92a such as a MEMS (Micro-Electro-Mechanical Systems) mirror or a galvanometer mirror is disposed on an optical path of the light from excitation light source 92. Excitation light L1 emitted from excitation light source 92 is incident on irradiation direction changing element 92a, and then reflected by irradiation direction changing element 92a to reach irradiation point LR1, so that photostimulable light R1 is emitted. In this case, excitation light L1 includes pre-reflection excitation light L1a and post-reflection excitation light L1b.

Irradiation direction changing element 92a can be driven by a drive source, and can change the position of the destination to which the incident excitation light L1 is reflected. For example, irradiation direction changing element 92a can change, in sub-scanning direction A2, the destination to which excitation light L1 is reflected. It is conceivable that irradiation direction changing element 92a may be driven in various modes such as an electromagnetic driving mode, a piezoelectric mode, and an electrostatic mode.

With such a configuration, excitation light source 92 itself does not move in the sub-scanning direction inside reading unit 90, but allows for movement of excitation light L1 in sub-scanning direction A2 for scanning. It may be considered that excitation light source 92 and irradiation direction changing element 92a constitute a light source that irradiates imaging plate 10 with excitation light L1, specifically, post-reflection excitation light L1b.

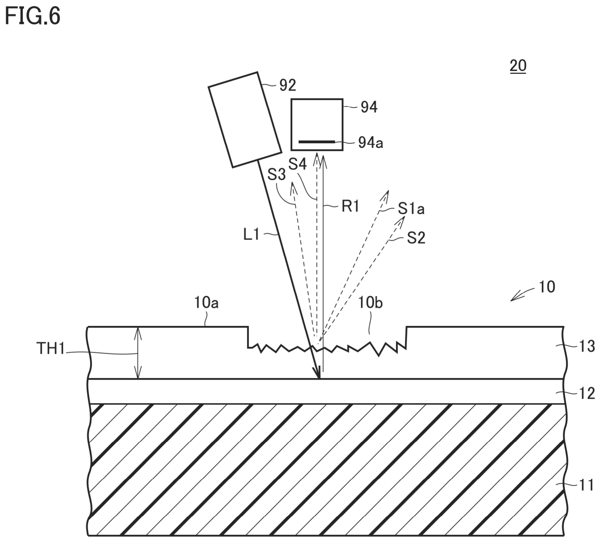

Next, Fig. 6 is a diagram for illustrating reflection of the light received on a surface in the second reflective area (information region 10b) of imaging plate 10. In image scanner 20, in order to read the radiographic image recorded on imaging plate 10 also in information region 10b, imaging plate 10 is irradiated with excitation light L1 from excitation light source 92. By excitation light L1 that has passed through protective layer 13 of imaging plate 10 and reached the surface of radiographic image forming layer 12, photostimulable light R1 is emitted based on the radiographic image recorded in radiographic image forming layer 12. By detecting photostimulable light R1, light detector 94 can read a radiographic image also in information region 10b.

However, information region 10b is provided with recess 14 having bottom surface 14a that is rougher than the surface of protective layer 13. Thus, the light reflected on the surface of protective layer 13 (information region protective layer 13b), specifically, the light reflected on bottom surface 14a of recess 14, includes not only the light (indicated as "Sla" in the figure) reflected in the same direction as the direction of reflected light S1 reflected on the surface of protective layer 13 (recording region protective layer 13a), but also the light (for example, reflected light S2, reflected light S3, and reflected light S4) reflected in a direction different from that of reflected light S1. Among rays of light reflected in directions different from the direction in which reflected light S1a is reflected, reflected light S4 is reflected in a direction along which it can be detected by light detector 94. Thus, light detector 94 can detect not only photostimulable light R1 but also reflected light S4 in information region 10b as shown in Fig. 6. Even if photostimulable light R1 emitted from radiographic image forming layer 12 is scattered to some extent in recess 14 (bottom surface 14a), detection surface 94d of light detector 94 exhibits two-dimensional broadening, which allows surface integration of the intensity of the light received in the detection surface area, with the result that dissipation is less likely to occur and a radiographic image is sufficiently formed. Preferably, detection surface 94d has a width in the forward sub-scanning direction that is equal to or greater than the width of imaging plate 10 set in the state in which imaging plate 10 is to be read, the width of imaging plate 10 being a width in the forward sub-scanning direction that has a maximum width in the forward sub-scanning direction. With such a configuration, light detector 94 can detect light without moving in the forward sub-scanning direction inside reading unit 90. If the numerical value of the distance between the front and back surfaces of protective layer 13, i.e., the thickness of protective layer 13, is TH1, the numerical value of the depth of the recess needs to be less than TH1.

In other words, in imaging plate 10, protective layer 13 has: recording region 10a (the first reflective area) in which the light received on its surface is reflected in the direction of reflected light S1 (the first direction); and information region 10b (the second reflective area) in which the light received on its surface is reflected in the direction of reflected light S4 (the second direction) that is different from the direction of reflected light S1. More specifically, in imaging plate 10, protective layer 13 has: recording region protective layer 13a (the first reflective area) in which the light received on its surface is reflected in the direction of reflected light S1 (the first direction); and information region protective layer 13b (the second reflective area) in which the light received on its surface is reflected in the direction of reflected light S4 (the second direction) that is different from the direction of reflected light S1. Thus, since light detector 94 can detect the presence or absence of recess 14 provided in information region 10b, the identification information of imaging plate 10 can be read based on the pattern of recess 14 formed in information region 10b. Light detector 94 is provided with optical filter 94a through which rays of light having wavelengths of photostimulable light R1 and reflected light S1 to reflected light S4 can pass.

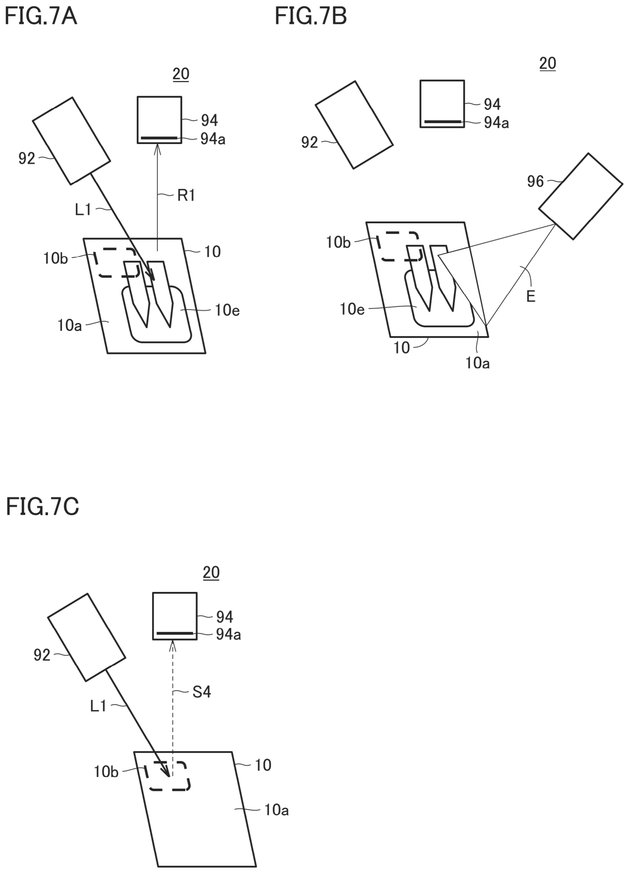

Specifically, a process of reading the recorded radiographic image and the identification information from imaging plate 10 will be hereinafter described. Figs. 7A to 7C are diagrams each for illustrating a process of reading the recorded radiographic image and the identification information from imaging plate 10 by image scanner 20 according to the embodiment. First, as shown in Fig. 7A, in image scanner 20, in order to read a radiographic image recorded on imaging plate 10, imaging plate 10 is irradiated with excitation light L1 from excitation light source 92. In imaging plate 10, photostimulable light R1 is emitted by excitation light L1 based on radiographic image 10e recorded in radiographic image forming layer 12. By detecting photostimulable light R1, light detector 94 can read radiographic image 10e. Note that light detector 94 detects only photostimulable light R1 in recording region 10a, but detects not only photostimulable light R1 but also the reflected light in information region 10b. However, since the reflected light is weaker than photostimulable light R1, it is difficult for image scanner 20 to extract only the reflected light from the rays of light detected by light detector 94.

Thus, after image scanner 20 reads radiographic image 10e by irradiation of the entire radiographic image forming layer 12 with excitation light L1, as shown in Fig. 7B, imaging plate 10 is irradiated with erasing light E from an erasing light source 96 to thereby erase radiographic image 10e recorded in radiographic image forming layer 12. Erasing light source 96 can irradiate imaging plate 10 with erasing light E for erasing radiographic image 10e from imaging plate 10. Erasing light E is, for example, visible light. Erasing light E may be white light, red visible light, or visible light of a different color. Erasing light source 96 may be a light emission diode (LED), a halogen lamp, or another light source.

Further, as shown in Fig. 7C, in image scanner 20, excitation light L1 is emitted from excitation light source 92 and applied to imaging plate 10 from which radiographic image 10e has been erased. Since no radiographic image is recorded on imaging plate 10, photostimulable light R1 is not emitted even when excitation light L1 is applied to imaging plate 10. Thus, light detector 94 can detect only reflected light S4 in information region 10b. From reflected light S4 detected by light detector 94, image scanner 20 can read the pattern of recess 14 formed in information region 10b and read out the identification information of imaging plate 10. After the entire radiographic image forming layer 12 is irradiated with excitation light L1, most of the latent image of the radiographic image is erased due to emission of photostimulable light. Thus, without irradiation with erasing light E, imaging plate 10 is further irradiated with excitation light L1 to thereby allow for detection of reflected light S4 in information region 10b. Herein, the state in which the latent image emits photostimulable light and energy is not accumulated any more is referred to as exhaustion. In Fig. 7C, at least information region 10b in the exhaustion state or the exhaustion area of information region 10b is irradiated with excitation light L1 to thereby detect reflected light S4.

As described above, the image data of the radiographic image acquired in Fig. 7A also includes image data of reflected light S4 reflected in information region 10b. Reflected light S4 reflected in information region 10b is weaker than photostimulable light R1 and has less influence on the image data of the radiographic image. However, in the case where image data of a clearer radiographic image is obtained by excluding the image data of reflected light S4 reflected in information region 10b, circuit unit 43 (the image processing unit) performs a subtraction process of subtracting the image data of the reflection image of reflected light S4 reflected in information region 10b and acquired in Fig. 7C from the image data of the radiographic image acquired in Fig. 7A.

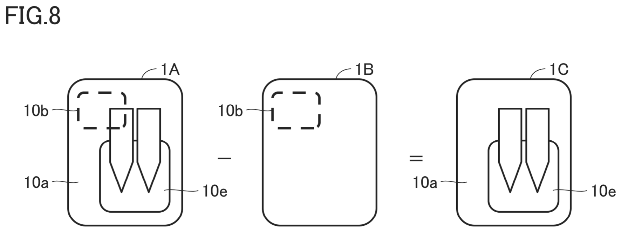

Fig. 8 is a diagram for illustrating image processing on an image obtained by reading the recorded radiographic image from imaging plate 10. As shown in Fig. 8, circuit unit 43 (the image processing unit) subtracts, from image data 1A of radiographic image 10e, image data 1B of the reflection image of reflected light S4 reflected in information region 10b to obtain image data 1C of only radiographic image 10e (a subtraction process). This makes it possible to obtain image data 1C of only radiographic image 10e that is not influenced by reflected light S4 reflected in information region 10b.

### (Modifications)

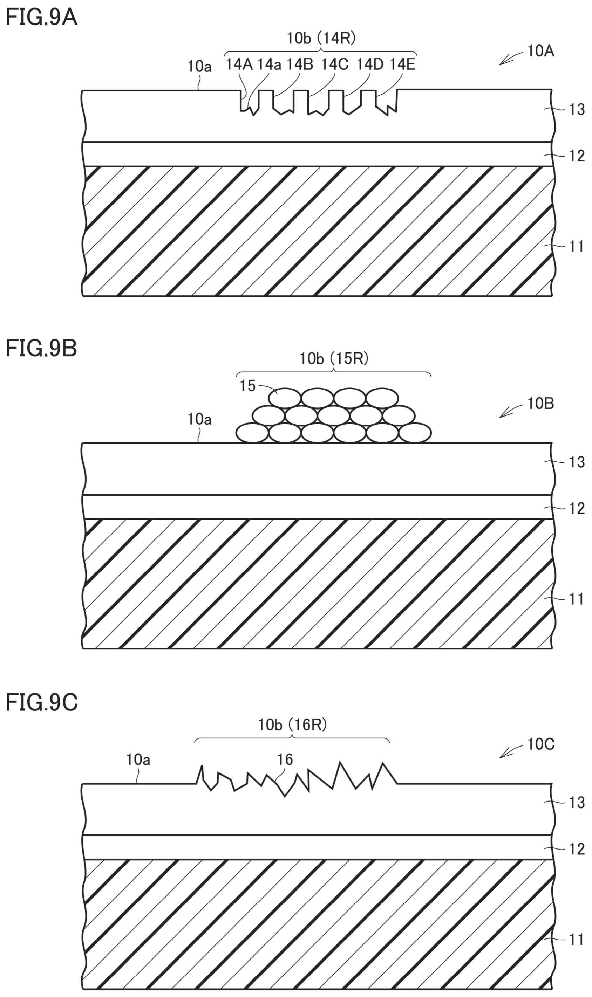

(1) In the configuration described in the above embodiment, recess 14 is provided in information region 10b and the identification information of imaging plate 10 is recorded. However, the configuration provided in information region 10b is not limited to recess 14 having a U-shaped cross-section as long as the configuration allows the received light to be reflected in a direction different from the direction in which the light received on the surface of protective layer 13 is reflected. Figs. 9A to 9C are schematic diagrams showing modifications of the second reflective area of the imaging plate.

First, in an imaging plate 10A shown in Fig. 9A, not one recess 14 but a plurality of recesses 14A to 14E are provided in information region 10b (information region protective layer 13b). Note that bottom surface 14a of each of the plurality of recesses 14A to 14E is rougher than the surface of protective layer 13. A region occupied by the plurality of recesses 14A to 14E may be referred to as a recess group portion 14R. Thus, the light reflected on bottom surface 14a of each of the plurality of recesses 14A to 14E includes not only the light reflected in the same direction as the direction of reflected light S1 reflected on the surface of protective layer 13, but also the light reflected in a direction different from that of reflected light S1. Image scanner 20 can read the identification information of imaging plate 10A from the pattern formed by a combination of the plurality of recesses 14A to 14E provided in information region 10b. When the identification information is recorded in information region 10b, it is necessary to form the pattern on the surface of protective layer 13 in a single stroke in order to form the pattern of the identification information in one recess 14. However, when the pattern of the identification information is formed by a combination of the plurality of recesses 14A to 14E, the degree of freedom of the pattern formed on the surface of protective layer 13 increases.

Further, in an imaging plate 10B shown in Fig. 9B, recess 14 is not provided in information region 10b (information region protective layer 13b), but fine particles of resin 15 are placed on information region 10b (information region protective layer 13b). A region occupied by the particles of resin 15 may be referred to as a particle group portion 15R. Resin 15 has properties of transparency to excitation light L1. Thus, also in information region 10b on which fine particles of resin 15 are placed, excitation light L1 reaches the surface of radiographic image forming layer 12, and photostimulable light R1 is emitted based on the radiographic image recorded in radiographic image forming layer 12. However, the fine particles of resin 15 make it possible not only to reflect the light in the same direction as the direction of reflected light S1 reflected on the surface of protective layer 13 but also to reflect the received light in a direction different from that of reflected light S1. Thus, image scanner 20 can read the identification information of imaging plate 10B by the pattern of the fine particles of resin 15 placed on information region 10b.

Further, in an imaging plate 10C shown in Fig. 9C, information region 10b (information region protective layer 13b) is not provided with recess 14 but is finely scratched on its surface to be processed into a rough surface 16. A region occupied by rough surface 16 may be referred to as a rough surface portion 16R. Like bottom surface 14a of recess 14, rough surface 16 makes it possible not only to reflect the light in the same direction as the direction of reflected light S1 reflected on the surface of protective layer 13, but also to reflect the light in a direction different from that of reflected light S1. Thus, image scanner 20 can read the identification information of imaging plate 10C by the pattern of rough surface 16 provided in information region 10b. Even if the photostimulable light emitted from radiographic image forming layer 12 is scattered to some extent in recess group portion 14R, particle group portion 15R, and rough surface portion 16R, detection surface 94d of light detector 94 exhibits two-dimensional broadening, which allows surface integration of the intensity of the light received in the detection surface area, with the result that dissipation is less likely to occur and a radiographic image is sufficiently formed.

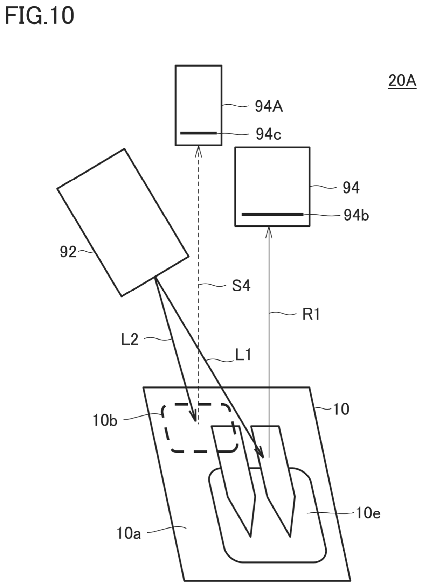

(2) In the description in the above embodiment, image scanner 20 detects, through use of one light detector 94, photostimulable light R1 emitted in recording region 10a and reflected light S4 in information region 10b. However, the light detector for detecting photostimulable light R1 and the light detector for detecting reflected light S4 do not need to be the same light detector. Fig. 10 is a schematic diagram showing a modification of the image scanner for reading imaging plate 10. As to an image scanner 20A shown in Fig. 10, configurations different from those of image scanner 20 shown in Fig. 4 will be hereinafter described, and other configurations are the same as the configurations of image scanner 20 described in the embodiment.

Image scanner 20A includes: excitation light source 92 that emits excitation light to imaging plate 10; light detector 94 (a first detector) that receives photostimulable light R1 generated from imaging plate 10 upon receipt of excitation light L1 emitted from excitation light source 92; and a reflected light detector 94A (a second detector) that receives reflected light S4 reflected in information region 10b (the second reflective area) upon receipt of excitation light L2. Light detector 94 is provided with an optical filter 94b through which light having a wavelength of photostimulable light R1 can pass. Reflected light detector 94A is provided with an optical filter 94c through which light having a wavelength of reflected light S4 can pass.

From photostimulable light R1 received by light detector 94, circuit unit 43 (the image processing unit) reads radiographic image 10e recorded on imaging plate 10. Further, from reflected light S4 reflected in information region 10b and received by reflected light detector 94A, circuit unit 43 (the image processing unit) reads the identification information of imaging plate 10 recorded in protective layer 13.

Since image scanner 20A detects photostimulable light R1 through use of light detector 94 and also detects reflected light S4 reflected in information region 10b through use of reflected light detector 94A, it can read the identification information of imaging plate 10 without erasing radiographic image 10e as described in Figs. 7A to 7C. Instead of providing two light detectors, i.e., light detector 94 and reflected light detector 94A, optical filter 94b through which light having a wavelength of photostimulable light R1 passes and optical filter 94c through which light having a wavelength of reflected light S4 passes may be set to be switchable in one light detector.

In the description in the above embodiment, the identification information of imaging plate 10 is recorded in information region 10b, but the information to be recorded in information region 10b is not limited to the identification information of imaging plate 10. In addition to the identification information of imaging plate 10, various pieces of information such as a manufacturing date, specifications, an expiration date, and a user name of imaging plate 10 can be recorded in information region 10b. It is sufficient that prescribed information can be recorded in information region 10b.

In the description in the above embodiment, recess 14 is formed by a laser marking device, but without being limited thereto, recess 14 may be formed by another device or another method. For example, recess 14 may be formed by physically cutting the surface of protective layer 13 with a blade or the like. Note that the depth of recess 14 needs to be limited to a depth at which the surface of radiographic image forming layer 12 is not exposed.

In the example described in the above embodiment, as shown in Figs. 1A and 1B, information region 10b is formed in a peripheral edge portion of protective layer 13, without being limited thereto. For example, information region 10b may be formed in a central portion of protective layer 13 or may be formed on the entire surface of protective layer 13. Even if recess 14 is formed in the entire surface of protective layer 13 and prescribed information is recorded thereby, the excitation light from excitation light source 92 reaches the surface of radiographic image forming layer 12 to emit photostimulable light, so that radiographic image 10e can be read out from imaging plate 10.

If the information recorded in information region 10b includes the identification information of imaging plate 10, circuit unit 43 can count the number of uses of imaging plate 10 based on the identification information that has been read. Since imaging plate 10 deteriorates every time it is used, it needs to be discarded after it is used a prescribed number of times. Imaging plate 10 may be configured such that a threshold value of the number of uses of imaging plate 10 can be set in advance. Then, when the number of uses reaches the threshold value, warning information may be generated. By recording the identification information of imaging plate 10 in information region 10b, circuit unit 43 can manage the number of uses of imaging plate 10, so that high-quality intraoral imaging can be implemented.

Although the embodiment of the present disclosure has been described, it should be understood that the embodiment disclosed herein is illustrative and non-restrictive in every respect. The scope of the present disclosure is defined by the terms of the claims and is intended to include any modifications within the scope and meaning equivalent to the terms of the claims.

## Claims

1. An imaging plate on which a radiographic image is recorded, the imaging plate comprising:
a substrate;
a radiographic image forming layer formed on the substrate, the radiographic image forming layer being configured to accumulate energy of a radiation irradiated onto the imaging plate, and the radiographic image forming layer being configured to emit emission light according to the energy accumulated; and
a protective layer formed on the radiographic image forming layer to protect the radiographic image forming layer, wherein
the protective layer has
a first reflective area having a surface on which light is received and reflected in a first direction, and
a second reflective area having a surface on which the light is received and reflected in a second direction different from the first direction, and
prescribed information is recorded in the protective layer by a pattern formed in the second reflective area.

2. The imaging plate according to claim 1, wherein the pattern formed in the second reflective area is formed by at least one recess provided in the surface of the protective layer.

3. The imaging plate according to claim 1 or 2, wherein the pattern formed in the second reflective area is formed by a combination of a plurality of recesses provided in the surface of the protective layer.

4. The imaging plate according to claim 2 or 3, wherein the recess is formed by a laser marking device.

5. The imaging plate according to any one of claims 1 to 4, wherein the prescribed information recorded in the protective layer includes identification information of the imaging plate.

6. The imaging plate according to any one of claims 1 to 5, wherein the second reflective area is formed in a peripheral edge portion of the protective layer.

7. An image scanner that reads a radiographic image from the imaging plate according to any one of claims 1 to 6, the image scanner comprising:
a light source configured to irradiate the imaging plate with excitation light;
a first detector configured to receive photostimulable light generated from the imaging plate upon receipt of the excitation light emitted from the light source;
a second detector configured to receive the excitation light reflected in the second reflective area; and
a circuit configured to read, from the photostimulable light received by the first detector, a radiographic image recorded on the imaging plate, wherein
the circuit is configured to read, from the excitation light reflected in the second reflective area and received by the second detector, the prescribed information recorded in the protective layer.

8. The image scanner according to claim 7, wherein the first detector and the second detector are the same.

9. The image scanner according to claim 7 or 8, wherein the first detector includes an optical filter through which wavelengths of the photostimulable light and the excitation light pass.

10. The image scanner according to any one of claims 7 to 9, further comprising an erasing light source configured to irradiate the imaging plate with erasing light for erasing a radiographic image recorded on the imaging plate, wherein
the circuit is configured to
receive, by the second detector, the excitation light reflected in the second reflective area of the imaging plate from which the recorded radiographic image has been erased by the erasing light source, and
read, from the excitation light received by the second detector, the prescribed information recorded in the protective layer.

11. The image scanner according to claim 10, wherein the circuit is configured to perform a subtraction process of subtracting, from a radiographic image obtained from the photostimulable light received by the first detector, a reflection image obtained from the excitation light received by the second detector after the recorded radiographic image is erased by the erasing light source.

12. The image scanner according to any one of claims 7 to 11, wherein the circuit is configured to, when the prescribed information includes identification information of the imaging plate, count a number of uses of the imaging plate based on the identification information that has been read.

13. A recording device for recording prescribed information on a protective layer of an imaging plate on which a radiographic image is recorded, the imaging plate including a substrate, a radiographic image forming layer formed on the substrate, the radiographic image forming layer being configured to accumulate energy of a radiation irradiated onto the imaging plate, and the radiographic image forming layer being configured to emit emission light according to the energy accumulated, and a protective layer formed on the radiographic image forming layer to protect the radiographic image forming layer, wherein the protective layer includes: a first reflective area having a surface on which light is received and reflected in a first direction, and a second reflective area having a surface on which the light is received and reflected in a second direction different from the first direction, and prescribed information is recorded in the protective layer by a pattern formed in the second reflective area, the recording device comprising:
a laser irradiator comprising a laser source to irradiate a laser light,
laser irradiation control circuitry configured to control the laser irradiator to shave a surface of protective layer with the laser light to form a recess and to form the prescribed information as the pattern indicating identification information of the imaging plate.
